# EUROPÄISCHE PATENTSCHRIFT

(11) **EP 3 727 211 B1**
(45) Veröffentlichungstag und Bekanntmachung des Hinweises auf die Patenterteilung: **27.07.2022**
(21) Anmeldenummer: 18826305.7
(22) Anmeldetag: 18.12.2018
(51) Int. Cl.: A61F 2/68, A61F 2/70

(54) **SYSTEM AUS ZUMINDEST EINER ORTHOPÄDIETECHNISCHEN KOMPONENTE UND EINER BEDIEN- UND/ODER FEEDBACKEINRICHTUNG**
SYSTEM OF AT LEAST ONE ORTHOPEDIC COMPONENT AND ONE OPERATING AND / OR FEEDBACK DEVICE
SYSTÈME D'AU MOINS UN COMPOSANT ORTHOPÉDIQUE ET UN DISPOSITIF DE FONCTIONNEMENT ET / OU DE RÉTROACTION

(30) Priorität: 22.12.2017 DE 102017131196
(43) Veröffentlichungstag der Anmeldung: 28.10.2020
(73) Patentinhaber: Otto Bock Healthcare Products GmbH, 1110 Wien (AT)
(72) Erfinder: PAPPE, Alexander, 1180 Wien (AT); WEIGL-POLLACK, Andreas, 3464 Goldgeben (AT); NOLTE, Michael, 37136 Seeburg (DE); ALBRECHT-LAATSCH, Erik, 37124 Rosdorf (DE); KAITAN, Robert, 1220 Wien (AT); HOFFMANN, Robert, 1160 Wien (AT); PAUSER, Thomas, 7035 Steinbrunn (AT); SCHRAMEL, Andreas, 1160 Wien (AT); SAGMEISTER, Luis, 2823 Pitten (AT)
(74) Vertreter: Gramm, Lins & Partner Patent- und Rechtsanwälte PartGmbB
(86) Internationale Anmeldenummer: PCT/EP2018/085656
(87) Internationale Veröffentlichungsnummer: WO 2019/121786

(56) Entgegenhaltungen:
- WO-A1-98/25552
- WO-A2-01/17466
- DE-B3-102016 114 075
- US-A1- 2014 276 298
- US-A1- 2016 302 686

## Beschreibung

Die Erfindung betrifft ein System aus zumindest einer orthopädietechnischen Komponente mit zumindest einem Energiespeicher und mehreren elektrischen und/oder elektronischen Einrichtungen.

Orthopädietechnische Komponenten sind Orthesen, Prothesen, Rollstühle, Datenlogger, Funkmodule, Akkus und Teile von Orthesen, Prothesen und Rollstühlen, beispielsweise Prothesen- oder Orthesenkniegelenke, Prothesenfüße, Rohradapter, Prothesenhände, Prothesen- oder Orthesenellenbogengelenke, Drehadapter, Prothesenschäfte, Schienen und andere Gelenke sowie Befestigungseinrichtung zum Festlegen von Orthesen oder Prothesen an einem Patienten.

An einer solchen orthopädietechnischen Komponente kann zumindest eine elektrische und/oder elektronische Einrichtung vorgesehen, beispielsweise um Daten aufzunehmen, Daten widerzugeben oder zu versenden. Über die elektrische und/oder elektronische Einrichtung kann ein Dämpfer eingestellt werden oder sie kann vorgesehen und eingerichtet sein, einen Antrieb zu realisieren um Verlagerungen oder Verschwenkungen von Komponenten zueinander zu bewirken oder um Daten zu verarbeiten, beispielsweise in Gestalt eines Mikroprozessors. Um diese elektrischen und/oder elektronischen Einrichtungen gegebenenfalls mit Energie versorgen zu können, ist der zumindest ein orthopädietechnischen Komponente zumindest ein Energiespeicher zugeordnet, damit Daten gespeichert oder ausgelesen werden können, eine Funkverbindung hergestellt werden kann, Antriebe mit Energie versorgt werden oder andere Aktionen ausgeführt werden können.

Die WO 98/25552 A1 betrifft eine Vorrichtung zum Versorgen einer Person mit Reizen, die einer externen Operation einer Prothese entsprechen, wobei die Operation der Prothese mit mehreren Sensoren erfasst wird, die an verschiedenen Stellen der Prothese angeordnet sind. Jeder Sensor hat eine eigene Sensorcharakteristik, so dass eine Vielzahl von Sensorcharakteristiken erzeugt wird. Jedem Sensor ist ein Ausgabeelement in Gestalt eines elektrischen Kontaktes zugeordnet, über das ein dem Sensorsignal entsprechendes Ausgabesignal abgegeben wird.

Die WO 01/17466 A2 betrifft eine Prothese der unteren Extremität mit einer Einrichtung zum Festlegen an einer Hüfte, an einem mit Becken mit einem künstlichen Hüftgelenk, einem Oberschenkelrohr, einem künstlichen Kniegelenk sowie einem Unterschenkelteil mit Prothesenfuß. Ein Antrieb ist dem Hüftgelenk zugeordnet, um anpassbar ein Flexionsmoment aufzubringen. Eine Energiespeichereinrichtung ist in einem proximalen Bereich der Prothese angeordnet und speichert elektrische Energie, die während der Standphase erzeugt wurde. Das Aktivieren und Deaktivieren des Antriebes wird über eine Steuerungseinrichtung kontrolliert. Eine Bedieneinheit ist über eine Antenne mit einer Empfängereinheit gekoppelt.

Die US 2014/0276298 A1 betrifft eine Vorrichtung und ein Verfahren zur Stimulation von Muskeln über elektrische Impulse mit einer Steuerungseinrichtung, die einer Batterie und eine Platine aufweist, auf der Mikroprozessoren angeordnet sind. Sensoren sind auf der Innenseite einer Manschette angeordnet und stehen im unmittelbaren Kontakt zu der Hautoberfläche. Zwei oder mehr Sensoren sind dazu vorgesehen, um den Energieverbrauch zu ermitteln.

Die US 2016/0302686 A1 betrifft Systeme, Vorrichtungen und Verfahren zum Steuern von prothetischen oder orthetischen Vorrichtungen auf der Grundlage elektromyographischer Signale. Ein Steuerungssystem kann unter anderem ein Sensormodul, ein Prothesenknöchelgelenk, eine zentrale Steuerungseinrichtung, eine Speichereinheit, ein Interface, ein Steuerungsantriebsmodul, einen Aktuator sowie ein Energiemodul enthalten.

Die DE 10 2016 114 075 B3 betrifft ein orthopädietechnisches System mit zumindest einer Stimulationselektrode zur Aktivierung zumindest eines Muskels. Das orthopädietechnische System kann eine orthetische oder prothetische Einrichtung aufweisen, die an einem Körper eines Patienten anlegbar und festlegbar ist. Die Einrichtung weist zumindest ein Gelenk auf, zwischen dessen proximaler und distaler Komponente eine verstellbare Widerstandseinrichtung angeordnet ist. Sensoren dienen zur Erfassung von Kräften, Positionen, Beschleunigungen und/oder Momenten. In Abhängigkeit von Sensorwerten wird die Widerstandseinrichtung verstellt.

Insbesondere bei einem System aus mehreren orthopädietechnischen Komponenten, beispielsweise einem Prothesenkniegelenk und einem Prothesenknöchelgelenk mit dazwischen angeordneten weiteren Komponenten, die beispielsweise Sensoren aufweisen oder als Betätigungseinrichtungen ausgebildet sind, um die Widerstände oder Antriebe in den jeweiligen Prothesengelenken zu aktivieren, deaktivieren oder zu verändern, kann es schwierig sein, die elektrischen und/oder elektronischen Einrichtungen zu überwachen.

Aufgabe der vorliegenden Erfindung ist es, ein System bereitzustellen, mit dem die zumindest eine orthopädietechnische Komponente mit mehreren elektrischen und/oder elektronischen Einrichtungen komfortabler bedient werden kann.

Erfindungsgemäß wird diese Aufgabe durch ein System mit den Merkmalen des Hauptanspruchs gelöst. Vorteilhafte Ausgestaltungen und Weiterbildungen der Erfindung sind in den Unteransprüchen, der Beschreibung sowie den Figuren beschrieben.

Das System aus zumindest einer orthopädietechnischen Komponente mit zumindest einem Energiespeicher und mehreren elektrischen und/oder elektronischen Einrichtungen sieht vor, dass zumindest eine Bedieneinrichtung und/oder eine Feedbackeinrichtung vorgesehen ist, die elektrischen und/oder elektronischen Einrichtungen zugeordnet und mit ihnen gekoppelt ist. Über die Bedien- und/oder Feedbackeinrichtung ist es möglich, alle elektrischen und/oder elektronischen Einrichtungen zu überwachen und anzusteuern oder einzustellen. So kann beispielsweise eine Ladezustandsanzeige aller Energiespeicher oder eine Funktionsfähigkeitsbestätigung aller elektrischen und/oder elektronischen Einrichtungen von der Feedbackeinrichtung erfolgen. Ebenso können sämtliche elektrischen und/oder elektronischen Einrichtungen über eine einzige Bedieneinrichtung aktiviert oder deaktiviert werden oder hinsichtlich ihres Betriebsmodusses eingeschaltet beziehungsweise umgeschaltet werden. Die Feedbackeinrichtung weist zumindest ein Feedbackelement auf, das als optische, taktile, akustische und/oder thermische Ausgabeeinrichtung ausgebildet ist. Damit können auf unterschiedliche Arten und Weisen die notwendigen oder angeforderten Informationen von der Feedbackeinrichtung bereitgestellt werden. Neben einer rein optischen Anzeige, beispielsweise auf einem Display, kann über eine akustische Ausgabeeinrichtung in Gestalt eines Lautsprechers entweder eine Sprachnachricht ausgegeben oder durch ein Signalton oder Abfolge von Signaltönen angezeigt werden, dass die orthopädietechnische Komponente aktiviert ist, deaktiviert ist oder in welchem Modus sie sich befindet. Solch eine Informationsausgabe kann durch eine taktile Ausgabeeinrichtung, beispielsweise eine Einrichtung zur Vibrationserzeugung unterstützt werden. Die taktile Ausgabeeinrichtung kann auch als alleiniges Feedbackelement ausgebildet sein. Über eine thermische Ausgabeeinrichtung kann ein weiterer Informationskanal für den Anwender bereitgestellt werden.

Die elektrische und/oder elektronische Einrichtung kann als Antrieb, Schalter, Solenoid, Datenverarbeitungseinrichtung und/oder Sensor ausgebildet sein. Als Antrieb ist insbesondere ein elektromotorischer Antrieb vorgesehen, grundsätzlich können neben rotatorischen Antrieben auch Linearantriebe vorgesehen sein. Über ein Solenoid können unterschiedliche Schaltzustände beispielsweise für Ventile in einem Hydraulik- oder Pneumatikkreislauf geschaltet oder in ihrer Position verändert werden. Neben aktiven Elementen, die eine Veränderung innerhalb des Systems bewirken, können auch Sensoren als elektrische und/oder elektronische Einrichtung eingesetzt werden, um Kräfte, Beschleunigung, Winkelstellungen von Komponenten zueinander, Momente, Geschwindigkeiten, Raumlagen oder auch Helligkeiten oder Umgebungslautstärken zu erfassen. Ebenfalls können Bewegungszyklen oder Belastungszyklen über die Sensoren erfasst werden, die dann der Bedien- und/oder Feedbackeinrichtung übermittelt werden, sodass eine einfache Auswertung in einer zentralen Bedien- und/oder Feedbackeinrichtung erfolgen kann.

Die Bedien- und/oder Feedbackeinrichtung ist bevorzugt mit dem zumindest einen Energiespeicher gekoppelt, um eine Trennung der elektrischen und/oder elektronischen Einrichtungen von dem Energiespeicher zu bewirken oder aufzuheben. Durch die Kopplung mit dem Energiespeicher ist es zudem möglich, dass die Feedbackeinrichtung eine aktive Signalausgabe bewirkt, beispielsweise eine optische Signalausgabe, eine akustische Signalausgabe oder ähnliches, um den Anwender, beispielsweise der Patient oder ein Orthopädietechniker, über den gegenwärtigen Zustand, die durchgeführten Belastungszyklen oder mögliche zukünftige Einstellungen zu informieren.

Die zumindest eine Bedieneinrichtung und/oder Feedbackeinrichtung ist in einer Weiterbildung der Erfindung reversibel an der orthopädietechnischen Komponente angebracht, beispielsweise formschlüssig über Schrauben, Klettverschlüsse, Klipse, Haken, Drehverschlüsse, Gurte, Laschen oder Taschen, in die die Bedien- und/oder Feedbackeinrichtung eingesetzt oder teilweise eingesetzt ist. Eine reversible kraftschlüssige Kopplung oder Befestigung kann insbesondere über eine Magnetkoppelung erreicht werden. Die reversible Befestigung kann auch über eine Kombination aus Formschluss und Kraftschluss erfolgen.

Die Bedieneinrichtung kann als Wärmesensor, Magnetfeldsensor, kapazitiver Sensor, optischer Sensor, Näherungssensor, radargestützter Bewegungssensor, Piezoelement und/oder einfacher elektrischer Schalter oder Kontakt ausgebildet sein, um auf verschiedene Arten und Weisen zu ermöglichen, dass die orthopädietechnische Komponente einfach und zuverlässig bedient werden kann. Über einen radargestützten Bewegungssensor kann beispielsweise über eine Handgestensteuerung die orthopädietechnische Komponente oder die orthopädietechnischen Komponenten des Systems bedient werden. Ebenso kann über optischen Sensor mit einer Näherungserkennung ermittelt werden, ob eine Bedienung stattfinden soll und wie die Art und Weise der Bewegung interpretiert werden soll. Über ein Piezoelement kann durch Drücken ein elektrisches Signal ausgelöst werden. Ein Wärmesensor kann einen Wärmeunterschied detektieren, wenn beispielsweise ein Finger oder ein anderes Körperteil auf den entsprechenden Sensor gelegt wird, um eine Eingabesignal an eine Steuerungseinrichtung auszugeben. Gleiches gilt für Magnetfeldsensoren oder kapazitive Sensoren, sodass über einen Touchscreen als Bedienelement die orthopädietechnische Komponente eingestellt, aktiviert oder deaktiviert werden kann.

Über das Feedbackelement kann neben der Darstellung der jeweils eingestellten Funktion und des Ladezustandes der Energiespeicher oder des Energiespeichers auch eine automatische Helligkeits- und/oder Lautstärkeregelung erfolgen. Dazu sind ein Helligkeitssensor und/oder ein Mikrofon an oder in der Feedbackeinrichtung vorgesehen, über die die Umgebungshelligkeit oder die Umgebungslautstärke gemessen werden. Über eine adaptive Helligkeits- und/oder Lautstärkeregelung wird dann eine Hintergrundbeleuchtung aktiviert oder deaktiviert oder die akustische Ausgabe an die Umgebungslautstärke angepasst.

Die Bedien- und/oder Feedbackeinrichtung kann Formschlusselemente und/oder Kraftschlusselemente zur Festlegung an der orthopädietechnischen Komponente aufweisen. Dadurch ist es möglich, die Bedien- und/oder Feedbackeinrichtung frei an der orthopädietechnischen Einrichtung, beispielsweise der Orthese, Prothese oder dem Rollstuhl zu positionieren und dort gegebenenfalls lösbar festzulegen. Als Formschlusselemente könnten Klipse, Klettverschlüsse, Haken oder auch Gewindeelemente vorgesehen sein, die eine dauerhafte und dennoch lösbare Verbindung an der orthopädietechnischen Komponente ermöglichen. Über Magnete kann die Bedien- und/oder Feedbackeinrichtung kraftschlüssig an der orthopädietechnischen Komponente festgelegt werden.

Nachfolgend werden Ausführungsbeispiele der Erfindung anhand der beigefügten Figuren näher erläutert. Es zeigen:
- Figur 1 -: eine erste Variante des Systems; sowie
- Figur 2 -: eine zweite Variante.

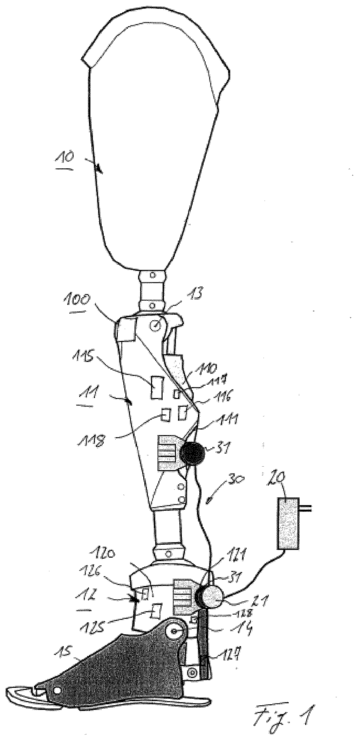

In der Figur 1 ist in einer schematischen Darstellung eine Prothese einer unteren Extremität mit mehreren orthopädietechnischen Komponenten 10, 11, 12, 15 dargestellt. Als erste orthopädietechnische Komponente 10 ist ein Oberschenkelschaft mit einem Anschlussadapter zur Befestigung an einem Oberteil eines Prothesenkniegelenkes gezeigt. An dem Prothesenkniegelenk ist eine zweite orthopädietechnische Komponente 11 schwenkbar um eine Schwenkachse 13 in Gestalt eines Unterschenkelteils angeordnet. An dem Unterschenkelteil als zweite orthopädietechnische Komponente 11 ist eine dritte orthopädietechnische Komponente 12 in Gestalt eines prothetischen Knöchelgelenks befestigt, an dem als vierte orthopädietechnische Komponente ein Prothesenfuß 15 schwenkbar um eine Schwenkachse 14 gelagert ist. Der Oberschenkelschaft als erste orthopädietechnische Komponente 10 ist eine reine passive Komponente und dient zur Aufnahme eines Oberschenkelstumpfes, um das Prothesenbein sicher an dem Patienten festlegen zu können. Über den Oberschenkelschaft und einen darin angeordneten Prothesenliner kann beispielsweise über ein Unterdrucksystem eine mechanische Festlegung des Prothesenbeines an dem Patienten erfolgen. Alternativ zu einer mehrteiligen Prothese kann das System als eine Orthese ausgebildet sein, ebenso kann als orthopädietechnische Komponente ein Rollstuhl oder eine prothetische oder orthetische Einrichtung für eine obere Extremität eingesetzt werden, beispielsweise ein Prothesenarm, eine Armorthese oder eine Schulterorthese. Als System werden auch Kombinationen von Prothesen, Orthesen und Rollstühlen oder anderen Mobilitätshilfen angesehen.

In dem dargestellten System ist in der zweiten orthopädietechnischen Komponente 11 eine erste elektrische und/oder elektronische Einrichtung 110 in Gestalt eines Hydraulikaktuators angeordnet. Der Hydraulikaktuator kann als passive Komponente ausgebildet sein und einen Hydraulikdämpfer aufweisen, der Stellantriebe aufweist, über die Ventile geöffnet oder geschlossen werden, um einen Extensionswiderstand und/oder Flexionswiderstand einstellen zu können. An dem Hydraulikaktuator kann zudem ein Speicher 115 zur Speicherung elektrischer Energie angeordnet oder diesem zugeordnet sein, um die Stellantriebe mit Energie zu versorgen. Bei einer Ausgestaltung der elektrischen und/oder elektronischen Einrichtung 110 als ein aktiver Aktuator ist eine Pumpeinrichtung oder ein mechanischer Energiespeicher, z.B. eine Feder oder Druckspeicher, dem Hydraulikdämpfer zugeordnet, über den es möglich ist, eine Bewegung des Oberschenkelschaftes relativ zu dem Unterschenkel zu bewirken oder zu unterstützen. Dazu wird die Energie aus dem Energiespeicher 115 in Bewegungsenergie umgewandelt, so dass beispielsweise eine Kolbenstange aus dem Hydraulikaktuator hinausbewegt wird, um eine Extensionsbewegung zu unterstützen oder zu bewirken. Umgekehrt kann durch eine Pumpe oder einen Druckspeicher Hydraulikfluid umgepumpt werden, so dass eine Kolbenstange in ein Gehäuse des Hydraulikaktuators eingefahren wird, damit sich der Abstand zwischen zwei Befestigungspunkten des Hydraulikaktuators an dem Oberteil und dem Unterteil des Prothesengelenkes verkürzt, um eine Flexionsbewegung auszuführen.

Distal zu der ersten elektrischen und/oder elektronischen Komponente 110 ist die zweite elektrische und/oder elektronische Komponente 120 in der orthopädietechnischen Komponente 12 in Gestalt eines Prothesenknöchelgelenkes angeordnet. Innerhalb des Prothesenknöchelgelenkes kann ebenfalls ein elektrischer und/oder hydraulischer Aktuator angeordnet sein, der über einen Speicher 125 für elektrische Energie mit elektrischer Energie versorgbar ist. Neben dem Speicher 125 für elektrische Energie ist in dem Prothesenknöchelgelenk eine Steuerungseinrichtung 126 angeordnet, an der Steuerungseinrichtung 116 in der ersten elektrischen und/oder elektronischen Einrichtung 110 angeordnet, um beispielsweise einen Antrieb 118, 128 zu aktivieren oder zu deaktivieren oder Daten eines Sensors 117, 127 zu verarbeiten, Sensordaten zu speichern und zur Steuerung weiterzuverwenden. In der Steuerungseinrichtung 116, 126 kann auch ein Datenspeicher sowie eine Verarbeitungsschaltung integriert sein.

Sowohl an der ersten orthopädietechnischen Komponente 11 als auch an der zweiten orthopädietechnischen Komponente 12 ist jeweils ein Versorgungsanschluss 111, 121 angeordnet, über den Energie und/oder Daten der jeweiligen elektrischen und/oder elektronischen Einrichtung 110, 120 zugeführt werden kann. Die Daten sowie die elektrische Energie können von einer Ladestation 20 auf den jeweiligen Versorgungsanschluss 111, 121 übertragen werden. Dazu ist an der Ladestation 20 ein Stecker 21 angeordnet, der mit dem jeweiligen Versorgungsanschluss 111, 121 kompatibel ist. In dem dargestellten Ausführungsbeispiel der Figur 1 wird der Stecker 21 der Ladestation 20 mit einem kaskadierenden Steckersystem einer Steckerverbindung 30 gekoppelt, die die beiden Versorgungsanschlüsse 111, 121 miteinander verbindet. An der Steckerverbindung 30 sind zwei Stecker 31 angeordnet, die auf einer Seite Kontakte aufweisen, die kompatibel zu den Kontakten der jeweiligen Versorgungsanschlüsse 111, 121 ausgebildet sind. Auf der den Versorgungsanschlüssen 111, 121 abgewandten Seite weisen die Stecker 31 Aufnahmen oder Buchsen auf, die mit den Kontakten des Steckers 21 der Ladestation 20 kompatibel sind.

Wird der Stecker 21 der Ladestation 20 auf die Rückseite eines Steckers 31 gesteckt, der über ein Kabel mit einem korrespondierenden Stecker 31 verbunden ist, so dass beide Versorgungsanschlüsse 111, 121 miteinander verbunden sind, können Energie und Daten von der Ladestation 20 sowohl auf die erste als auch auf die zweite elektronische und/oder elektrische Einrichtung 110, 120 übertragen werden. Dadurch werden die Speicher 115, 125 zur Speicherung elektrischer Energie aufgefüllt sowie die Steuerungseinrichtungen 116, 126 mit Daten wie Programmen, Steuerungsdaten, Softwareupdates oder dergleichen versorgt. Über das System aus Ladestation 20 mit Stecker 21, Versorgungsanschlüssen 111, 121 sowie die Steckerverbindung 30 mit den Steckern 31 ist es möglich, ein elektrisches Verbindungssystem für orthopädietechnische Komponenten 11, 12, insbesondere Orthesen, Prothesen und/oder Rollstühle, bereitzustellen, mit dem es möglich ist, die jeweiligen elektrischen und/oder elektronischen Einrichtungen 110, 120 aufzuladen, miteinander zu verbinden, um Energie aus den jeweiligen Energiespeichern 115, 125 untereinander zu verteilen oder um Steuerungsabläufe miteinander zu koordinieren. Über das System ist es möglich, nicht nur extern Daten aus der Ladestation 20 sowie elektrische Energie dem orthopädietechnischen System, beispielsweise der Orthese, Prothese oder dem Rollstuhl zuzuführen, sondern auch innerhalb der orthopädietechnischen Einrichtung zwischen den jeweiligen orthopädietechnischen Komponenten 11, 12 einen Energie- und/oder Datenaustausch zu ermöglichen.

Wenn in den unterschiedlichen Komponenten 11, 12 mit den verschiedenen elektrischen und/oder elektronischen Einrichtungen 110, 120 unterschiedliche maximal benötigte Leistungen in dem jeweiligen Verbraucher, beispielsweise Antrieb 118, 128, vorhanden sind, kann die jeweils maximal benötigte Leistung eines Verbrauchers 118, 128 oder einer Steuerungseinrichtung 116, 126 über einen elektrischen Widerstand kodiert werden. Durch die Kompatibilität der Versorgungsanschlüsse 111, 121 mit den jeweiligen Steckern 21, 31 ist es möglich, mehrere Ladeanschlüsse an einer orthopädietechnischen Komponente bereitzustellen, um es einem Anwender zu erleichtern, das orthopädietechnische Hilfsmittel mit einer Ladestation 20 zu koppeln. Dadurch kann der Nutzer den für ihn am besten zu erreichenden Versorgungsanschluss 111, 121 frei auswählen, so dass ein oder mehrere Anschlüsse zum zentralen Laden aller elektrischer und/oder elektronischer Einrichtungen 110, 120 vorhanden sind. Das Laden mit elektrischer Energie ebenso wie die Versorgung mit Daten kann gleichzeitig oder sequentiell erfolgen. Sind beispielsweise die elektrischen und/oder elektronischen Einrichtungen 110, 120 nicht über die Steckerverbindung 30 miteinander gekoppelt, können Daten und Energie über die Ladestation 20 nacheinander durch den Stecker 21 übertragen werden. Die jeweilige elektronische und/oder elektrische Einrichtung 110, 120 ist mit einer Kodierung versehen, so dass über die Ladestation 20 erkannt wird, welche Einrichtung gerade mit Energie und/oder Daten versorgt werden soll, so dass sowohl die richtige Energiemenge als auch die richtigen Daten übertragen werden.

Ist die Ladestation 20 nicht angeschlossen, kann über die Steckerverbindung 30 ein Daten- und Energieaustausch zwischen den Einrichtungen 110, 120 erfolgen. Ebenfalls ist es möglich, über eine Steckerverbindung 30 mehrere orthopädietechnische Hilfsmittel miteinander zu kombinieren, beispielsweise eine Orthese oder eine Prothese mit einem Rollstuhl, der beispielsweise über einen größeren Energiespeicher in Gestalt einer Batterie verfügt, so dass zur Aufrechterhaltung der Mobilität des Anwenders oder Patienten Energie aus dem Rollstuhl in die Orthese oder Prothese übertragen werden kann.

In dem dargestellten Ausführungsbeispiel gemäß der Figur 1 ist an dem Unterschenkelteil eine Bedien- und/oder Feedbackeinrichtung 100 angeordnet und an dem Unterschenkelteil 11 reversibel festgelegt, beispielsweise durch Kraftschlusselemente oder Formschlusselemente, über die es möglich ist, die Bedien- und/oder Feedbackeinrichtung 100 an der orthopädietechnischen Komponente 11 festzulegen und wieder abzunehmen. Die Formschlusselemente können als Stecker, Clipse, Klettverschlüsse, Klammern, Einsteckelemente oder auch Schrauben ausgebildet sein, als Kraftschlusselemente sind insbesondere Magnete geeignet, über die eine einfache kraftgesteuerte Festlegung und Entfernung der Bedien- und/oder Feedbackeinrichtung 100 erfolgen kann. Eine kraftschlüssige Kopplung hat den Vorteil, dass in der Regel eine beschädigungsfreie Trennung der Bedien- und/oder Feedbackeinrichtung 100 von der jeweiligen orthopädietechnischen Komponente erfolgen kann, sollte der Nutzer der Prothese, Orthese oder des Rollstuhls gegen einen Gegenstand stoßen. Darüber hinaus ist eine nahezu beliebige Positionierung an derjenigen Stelle möglich, die für den Anwender am geeignetsten erscheint, sofern ausreichend magnetische oder ferromagnetische Elemente an der orthopädietechnischen Einrichtung vorhanden sind. Über den Festlegungsbereich, beispielsweise über Stecker oder über Kontaktelemente, die sowohl an der Bedien- und/oder Feedbackeinrichtung 100 und der Befestigungsstelle an der jeweiligen orthopädietechnischen Komponente 10, 11, 12, 15 vorhanden sind, kann auch eine energetische sowie datenübertragende Kopplung zwischen der orthopädietechnischen Komponente mit dem angeordneten oder zugeordneten Energiespeicher 115, 125 und der jeweiligen elektrischen und/oder elektronischen Einrichtung 110 mit den Antrieben, Schaltern, Datenverarbeitungseinrichtungen und Sensoren erfolgen.

Die Bedien- und/oder Feedbackeinrichtung 100 kann für das Bedienteil einen Wärmesensor, Magnetfeldsensor und kapazitiven Sensor, optischen Sensor, Näherungssensor, radargestützten Bewegungssensor, ein Piezoelement und/oder einen elektrischen Kontakt aufweisen, um einem Nutzer der orthopädietechnischen Komponente oder des Systems aus einer orthopädietechnischen Komponente und der Bedien- und/oder Feedbackeinrichtung es auf einfache Art und Weise zu ermöglichen, die elektrischen und/oder elektronischen Einrichtungen mit den daran angeordneten, oder damit gekoppelten Bauteilen, Einrichtungen oder Vorrichtungen zu beeinflussen, insbesondere einzuschalten, auszuschalten oder Veränderungen in Einstellungen vorzunehmen. Bei der kombinierten Ausgestaltung mit einer Feedbackeinrichtung kann die Feedbackeinrichtung zumindest ein Feedbackelement aufweisen, das als eine optische, taktile, akustische oder thermische Ausgabeeinrichtung ausgebildet ist. Über eine optische Ausgabeeinrichtung wird der Nutzer der orthopädietechnischen Komponente mittels einer Anzeige darüber informiert, in welchem Zustand sich die orthopädietechnische Komponente oder die elektrische und/oder elektronische Einrichtung befindet, beispielsweise welche Widerstände in einer Dämpfereinrichtung vorhanden sind, wie der Ladezustand eines Energiespeichers ist, wie die Verteilung von Energiemengen in mehreren Energiespeichern ist, welche Softwareversion vorhanden ist, ob ein Wartungstermin vereinbart werden muss oder dergleichen. Eine taktile Ausgabeeinrichtung kann beispielsweise ein Vibrationssignal ausgeben, das eine Aktivierung oder Deaktivierung einer elektrischen und/oder elektronischen Komponente anzeigt. Über eine akustische Ausgabe kann beispielsweise mittels eines Signaltons oder einer Sprachausgabe dem Nutzer mitgeteilt werden, in welchem Modus sich die orthopädietechnische Komponente befindet oder in welchem Zustand eine Energiespeichereinrichtung ist.

Die Bedien- und/oder Feedbackeinrichtung 100 kann einen Helligkeitssensor aufweisen, beispielsweise um automatisch ein Bedienfeld zu beleuchten oder Einrichtungen an der orthopädietechnischen Komponente zu beleuchten oder zu dimmen. Jedenfalls kann ein Mikrophon mit der Bedien- und/oder Feedbackeinrichtung 100 gekoppelt oder darin ausgebildet sein, beispielsweise um eine adaptive Lautstärkeregelung bei einer Ausgabe mit einem Lautsprecher vorzunehmen oder aber um über das Mikrophon akustische Befehle über eine Spracheingabe einzugeben.

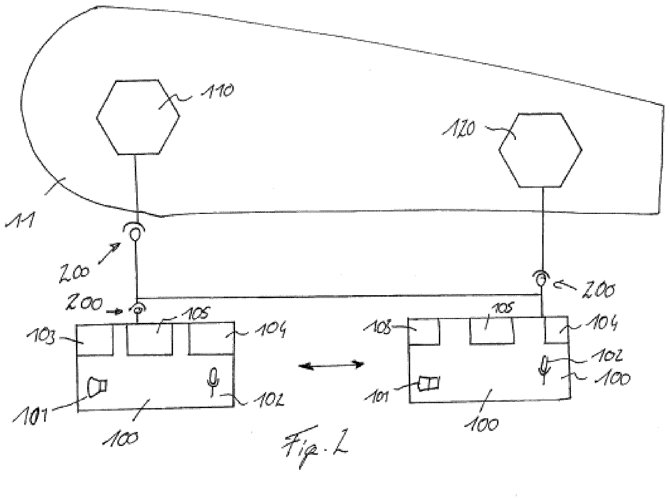

In der Figur 2 zeigt in einer schematischen Darstellung eine orthopädietechnische Komponente 11 gemäß der Figur 1 in Gestalt eines Unterschenkelteils mit einer ersten elektrischen und/oder elektronischen Einrichtung 110 als Hydraulikaktuator zum Beeinflussen einer Flexions- und Extensionsbewegung zwischen einem Oberschenkelschaft und einem Unterschenkelteil sowie einer zweiten elektrischen und/oder elektronischen Einrichtung 120 in Gestalt eines Prothesenknöchelgelenkes. An den elektrischen und/oder elektronischen Einrichtungen 110, 120 sind Kopplungseinrichtungen in Gestalt von Formschlusselementen und/oder Kraftschlusselementen 200 angeordnet, die gleichzeitig auch Einrichtungen zur elektrischen Kopplung sowie einer Datenverarbeitungskopplung aufweisen können. Alternativ zu einer mechanischen Kopplung über die Formschlusselemente 200, die beispielsweise auch als verriegelbare Stecker ausgebildet sein können, oder Kraftschlusselemente 200 mit zugeordneten Kontaktflächen zur Energie- und Datenübertragung kann insbesondere die Datenübertragung auch drahtlos erfolgen. Die Bedien- und/oder Feedbackeinrichtung 100 kann einen Lautsprecher 101 und ein Mikrophon 102 aufweisen, um eine akustische Ausgabe als Feedbackfunktion und eine akustische Eingabe über eine Sprachsteuerung bei einer Bedienfunktion ausüben zu können. Ebenfalls kann eine taktile Ausgabeeinrichtung 103 in Gestalt eines Vibrationsfeldes und/oder ein Display 105 sowie eine taktile Eingabeeinrichtung oder ein Wärmesensor, Magnetfeldsensor, optischer Sensor oder Näherungssensor oder auch elektrischer Kontakt 104 vorhanden sein, um entsprechende Eingabebefehle über die Bedien- und/oder Feedbackeinrichtung 100 in die orthopädietechnische Komponente 11 zur Steuerung der elektrischen und/oder elektronischen Einrichtung 110 vornehmen zu können. Über die eine Bedien- und/oder Feedbackeinrichtung 100 können beide elektrischen und/oder elektronischen Einrichtungen 110, 120 angesteuert und Rückkopplungssignale von diesen empfangen werden. Ebenfalls ist es möglich, eine zweite Bedien- und/oder Feedbackeinrichtung 100 vorzusehen, die der zweiten elektrischen und/oder elektronischen Einrichtung 120 zugeordnet ist. Auch hier kann die Kopplung über Formschluss- oder Kraftschlusselemente 200 mit integrierter elektrischer und datenübertragender Kopplung oder auch gegenenfalls ohne datenübertragende Kopplung erfolgen. Die beiden Bedien- und/oder Feedbackeinrichtungen 100 können drahtlos miteinander synchronisiert werden, so dass von jeder Bedien- und/oder Feedbackeinrichtung 100 alle elektrischen und/oder elektronischen Einrichtungen 110, 120 angesteuert werden können und von diesen Daten oder Rückmeldungen empfangen werden können. In der Figur 2 sind die beiden Bedien- und/oder Feedbackeinrichtungen 100 reversibel über die Kraft- und/oder Formschlusselemente 200 mit den elektrischen und/oder elektronischen Einrichtungen 110, 120 gekoppelt. Die rechte Bedien- und/oder Feedbackeinrichtung 100 ist dafür ausgebildet, an beiden Kraft- und/oder Formschlusselementen 200 reversibel festgelegt zu werden, um beide elektrischen und/oder elektronischen Einrichtungen 110, 120 bedienen oder von diesen Rückmeldungen erhalten und ausgeben zu können. Es ist auch vorgesehen, für jede elektrische und/oder elektronische Einrichtung 110, 120 eine Bedien- und/oder Feedbackeinrichtung 100 einzusetzen. In der dargestellten Kombination kann der Anschluss der linken Bedien- und/oder Feedbackeinrichtung 100 eine Zuordnung zu der linken elektrischen und/oder elektronischen Einrichtung 110 bewirken, die rechte Bedien- und/oder Feedbackeinrichtung 100 ist dann nur für die rechte elektrische und/oder elektronische Einrichtung 120 zuständig. In einer Variante können auch beide Bedien- und/oder Feedbackeinrichtungen 100 mit beiden elektrischen und/oder elektronischen Einrichtungen 110, 120 kommunizieren.

## Patentansprüche

1. System aus zumindest einer orthopädietechnischen Komponente (10, 11, 12, 15) mit zumindest einem Energiespeicher (115, 125) und mehreren elektrischen und/oder elektronischen Einrichtungen (110, 120) sowie zumindest einer Bedieneinrichtung und/oder Feedbackeinrichtung (100), die den elektrischen und/oder elektronischen Einrichtungen (110, 120) zugeordnet und mit ihnen gekoppelt ist, **dadurch gekennzeichnet, dass** die Feedbackeinrichtung (100) zumindest ein Feedbackelement (101, 103, 105) aufweist, das als optische, taktile, akustische und/oder thermische Ausgabeeinrichtung ausgebildet ist.

2. System nach Anspruch 1, **dadurch gekennzeichnet, dass** die elektrische und/oder elektronische Einrichtung (110, 120) als Antrieb (118, 128), Schalter, Solenoid, Datenverarbeitungseinrichtung (116, 126) und/oder Sensor (117, 127) aufweist.

3. System nach Anspruch 1 oder 2, **dadurch gekennzeichnet, dass** die Bedien- und/oder Feedbackeinrichtung (100) mit dem Energiespeicher (115, 125) gekoppelt ist.

4. System nach einem der voranstehenden Ansprüche, **dadurch gekennzeichnet, dass** die zumindest eine Bedieneinrichtung und/oder Feedbackeinrichtung (100) reversibel an der orthopädietechnischen Komponente (10, 11, 12, 15) angebracht ist.

5. System nach einem der voranstehenden Ansprüche, **dadurch gekennzeichnet, dass** die Bedieneinrichtung (100) einen Wärmesensor, Magnetfeldsensor, kapazitiver Sensor, optischer Sensor, Näherungssensor, radargestützter Bewegungssensor, Piezoelement und/oder elektrischer Kontakt (104) aufweist.

6. System nach einem der voranstehenden Ansprüche, **dadurch gekennzeichnet, dass** die Bedien- und/oder Feedbackeinrichtung (100) einen Helligkeitssensor und/oder Mikrophon (102) und eine adaptive Helligkeits- und/oder Lautstärkeregelung aufweist.

7. System nach einem der voranstehenden Ansprüche, **dadurch gekennzeichnet, dass** die Bedien- und/oder Feedbackeinrichtung (100) Formschlusselemente (200) und/oder Kraftschlusselemente zur Festlegung an einer orthopädietechnischen Komponente (10, 11, 12, 15) aufweist.

## Claims

1. A system made of at least one orthopedic component (10, 11, 12, 15) comprising at least one energy store (115, 125) and a plurality of electrical and/or electronic devices (110, 120) and at least one operator control device and/or feedback device (100), which is assigned to the electrical and/or electronic devices (110, 120) and coupled thereto, **characterized in that** the feedback device (100) has at least one feedback element (101, 103, 105), which is embodied as an optical, tactile, acoustic and/or thermal output device.

2. The system as claimed in claim 1, **characterized in that** the electrical and/or electronic device (110, 120) has, as a drive (118, 128), a switch, a solenoid, a data processing device (116, 126) and/or a sensor (117, 127).

3. The system as claimed in claim 1 or 2, **characterized in that** the operator control and/or feedback device (100) is coupled to the energy store (115, 125).

4. The system as claimed in any one of the preceding claims, **characterized in that** the at least one operator control device and/or feedback device (100) is reversibly attached to the orthopedic component (10, 11, 12, 15).

5. The system as claimed in any one of the preceding claims, **characterized in that** the operator control device (100) has a heat sensor, magnetic field sensor, capacitive sensor, optical sensor, proximity sensor, radar-assisted motion sensor, piezo element and/or electrical contact (104).

6. The system as claimed in any one of the preceding claims, **characterized in that** the operator control and/or feedback device (100) has a brightness sensor and/or a microphone (102) and adaptive brightness and/or volume control.

7. The system as claimed in any one of the preceding claims, **characterized in that** the operator control and/or feedback device (100) has form-fit elements (200) and/or force-fit elements for securing to an orthopedic component (10, 11, 12, 15).

## Revendications

1. Système composé d'au moins un composant orthopédique (10, 11, 12, 15) ayant au moins un accumulateur d'énergie (115, 125) et de plusieurs dispositifs électriques et/ou électroniques (110, 120) ainsi que d'au moins un dispositif de commande et/ou d'un dispositif de retour d'information (100) associé aux dispositifs électriques et/ou électroniques (110, 120) et couplé à ceux-ci,
**caractérisé en ce que** le dispositif de retour d'information (100) comporte au moins un élément de retour d'information (101, 103, 105) réalisé sous forme de dispositif de sortie optique, tactile, acoustique et/ou thermique.

2. Système selon la revendication 1,
**caractérisé en ce que** le dispositif électrique et/ou électronique (110, 120) comporte en tant qu'entraînement (118, 128), un commutateur, un solénoïde, un dispositif de traitement de données (116, 126) et/ou un capteur (117, 127).

3. Système selon la revendication 1 ou 2,
**caractérisé en ce que** le dispositif de commande et/ou de retour d'information (100) est couplé à l'accumulateur d'énergie (115, 125).

4. Système selon l'une des revendications précédentes,
**caractérisé en ce que** ledit au moins un dispositif de commande et/ou de retour d'information (100) est monté de manière réversible sur le composant orthopédique (10, 11, 12, 15).

5. Système selon l'une des revendications précédentes,
**caractérisé en ce que** le dispositif de commande (100) comprend un capteur thermique, un capteur de champ magnétique, un capteur capacitif, un capteur optique, un capteur de proximité, un capteur de mouvement assisté par radar, un élément piézoélectrique et/ou un contact électrique (104).

6. Système selon l'une des revendications précédentes,
**caractérisé en ce que** le dispositif de commande et/ou de retour d'information (100) comporte un capteur de luminosité et/ou un microphone (102) et un régulateur adaptatif de la luminosité et/ou du volume.

7. Système selon l'une des revendications précédentes,
**caractérisé en ce que** le dispositif de commande et/ou de retour d'information (100) présente des éléments de coopération de forme (200) et/ou des éléments de coopération de force pour l'immobilisation à un composant orthopédique (10, 11, 12, 15).
